# EUROPÄISCHE PATENTANMELDUNG

(11) **EP 0 984 014 A2**
(43) Veröffentlichungstag der Anmeldung: **08.03.2000**
(21) Anmeldenummer: 99114728.1
(22) Anmeldetag: 26.07.1999
(51) Int. Cl.: C07D 473/18

(54) **Verfahren zur Herstellung von Guanin**

(30) Priorität: 27.08.1998 DE 19839013
(71) Anmelder: Degussa-Hüls Aktiengesellschaft, 60287 Frankfurt am Main (DE)
(72) Erfinder: Theis, Christoph, Dr., 53859 Niederkassel (DE); Bruhn, Stefan, Dr., 21447 Handorf (DE)

(57) **Zusammenfassung**

Die vorliegende Erfindung betrifft ein Verfahren zur Herstellung von Guanin, wobei 2,4-Diamino-5-formylamino-6-hydroxypyrimidin (DAFHP) mit Ameisensäure in Abwesenheit von Formamid umgesetzt wird. Dieses Verfahren wird vorzugsweise mit Wasserzusatz und unter Rückflußtemperatur durchgeführt.

## Beschreibung

Die vorliegende Erfindung betrifft ein Verfahren zur Herstellung von Guanin (2-Amino-1,9-dihydropurin-6-on), ausgehend von 2,4-Diamino-5-formylamino-6-hydroxypyrimidin (DAFHP).

Die Nucleinsäurebase Guanin ist als wichtiges Zwischenprodukt zur Synthese pharmakologisch aktiver Verbindungen, insbesondere antiviraler Wirkstoffe, von großer Bedeutung. Guanin wird z.B. als Vorstufe für Acyclovir, das gemäß DE-A-35 44 461 zur Therapie von Virusinfektionen geeignet ist, benötigt.

Die Umsetzung von 4,5-Diaminopyrimidin-Sulfaten mit Formamid zu den entsprechenden Purinen ist literaturbekannt (Robins et al., J. Am. Chem. Soc. 75 (1953) 263). Zur Synthese des Guanins wird 2,4,5-Triamino-6-hydroxypyrimidin-Sulfat (TAHP-Sulfat) eingesetzt. Nach der DE-A-37 29 471 kann Guanin durch Erhitzen einer Suspension von TAHP-Sulfat in Formamid auf bis zu 200°C unter Abdestillation des gebildeten Reaktionswassers erhalten werden. Nachteilig hierbei ist die Eigenschaft des Formamids, sich bei den benötigten hohen Temperaturen teilweise zu zersetzen, was neben der Bildung von Kohlenmonoxid und Ammoniak verfärbte Guanin-Rohprodukte zur Folge hat, die einen hohen Reinigungsaufwand erfordern. Die Notwendigkeit, das in freier Form unbeständige TAHP in Form seires Sulfats einzusetzen, bedingt eine hohe Salzfracht, was einen weiteren Nachteil darstellt.

Gemäß der EP-A-0 415 028 wird Guanin durch Umsetzung von TAHP-Sulfat mit Alkaliformiat und Ameisensäure erhalten. Dieses Verfahren vermeidet zwar die mit dem Einsatz von Formamid verbundenen Nachteile, führt jedoch ebenfalls zu einem wirtschaftlich und ökologisch ungünstigen hohen Zwangsanfall an Salzfracht in Form von Alkalisulfat in der Reaktionsmischung.

Bei den genannten, auf TAHP-Sulfat basierenden Verfahren wird als Zwischenprodukt 2,4-Diamino-5-formylamino-6-hydroxypyrimidin (im folgenden als DAFHP bezeichnet) durchlaufen, das in situ zum Guanin umgesetzt wird.

Nach der DE-A-41 36 114 kann Guanin auch ausgehend von isoliertem DAFHP durch Erhitzen in Formamid auf mindestens 140°C gewonnen werden. Das Verhältnis von DAFHP zu Formamid betragt 1:2 bis 1:3. Der Reaktionsmischung kann bis zu 10% Ameisensäure zugesetzt werden. Das dabei eingesetzte DAFHP wird beispielsweise durch ein Verfahren nach der EP-A-0 267 594 erhalten, worin 2,4-Diamino-6-hydroxy-5-nitrosopyrimidin katalytisch hydriert und zu TAHP-Sulfat umgesetzt wird. Nach der Hydrierung wird das Reaktionsgemisch mit Ameisensäure, gegebenenfalls unter Zusatz einer Mineralsäure, versetzt, um DAFHP quantitativ zu erhalten. Das in der DE-A-41 36 114 beschriebene Verfahren ist zwar salzfrei, weist allerdings die bereits erwähnten Nachteile auf, die mit der Verwendung von Formamid (wie Zersetzung, aufwendige Reinigung des Endprodukts) einhergehen. Das nach der DE-A-41 36 114 gewonnene Guanin wird mit einem Gehalt (HPLC) von weniger als 98.0% erhalten. Nach der Reinigung kommt es daher zu Ausbeuteverlusten.

Es ist daher die Aufgabe der vorliegenden Erfindung, ein technisches Verfahren zur Herstellung von Guanin bereitzustellen, welches die genannten Nachteile des Standes der Technik nicht aufweist und bei dem Guanin in hoher Raum-Zeit-Ausbeute und Reinheit erhalten wird.

Diese Aufgabe wird erfindungsgemäß durch ein Verfahren zur Herstellung von Guanin ausgehend von 2,4-Diamino-5-formylamino-6-hydroxypyrimidin (DAFHP) gelöst, das dadurch gekennzeichnet ist, daß man isoliertes DAFHP in Abwesenheit von Formamid in Ameisensäure mit oder ohne Wasserzusatz unter Rückflußbedingungen zum Guanin umsetzt. Das isolierte DAFHP ist beispielsweise durch das in der EP-A-0267594 beschriebene Verfahren leicht zugänglich.

Bezogen auf DAFHP werden dabei bevorzugt bis zu 2 Äquivalente, besonders bevorzugt 0.5 bis 1.5 Äquivalente Wasser eingesetzt. Der Einsatz von mehr als 2 Äquivalenten Wasser bedingt einen erhöhten Reinigungsaufwand des resultierenden Guanins und ist daher nicht zweckmäßig. Die Reaktion wird bei Normaldruck und bei einer Temperatur, die sich einstellt, wenn wasserhaltige Ameisensäure unter Rückfluß siedet, durchgeführt.

Es hat sich überraschend und nicht vorhersehbar gezeigt, daß das erfindungsgemäße Verfahren den Einsatz hoher molarer DAFHP-Konzentrationen erlaubt, was zweifellos einen besonderen Vorteil darstellt. In der Ausführungsform ohne Wasserzusatz beträgt die Molalität (bezogen auf DAFHP) bis zu 2.5, bevorzugt 1.80 bis 2.25. Die Reaktionszeit betragt 12 bis 24 Stunden, bevorzugt 15 bis 18 Stunden. Längere Reaktionszeiten sind prinzipiell möglich, führen jedoch zu keiner signifikanten Verbesserung; zudem widersprechen sie dem erfindungsgemäßen Ziel einer hohen Raum-Zeit-Ausbeute. Kürzere Reaktionszeiten haben deutliche Ausbeuteverluste durch nicht umgesetztes DAFHP zur Folge. Es hat sich jedoch als besonders überraschend herausgestellt, daß die bevorzugte Ausführungsform mit Wasserzusatz nicht nur eine nochmalige deutliche Erhöhung der molaren DAFHP-Konzentration, sondern darüber hinaus auch eine deutliche Verkürzung der Reaktionszeit erlaubt. Die Molalität beträgt hierbei bis zu 5.0, bevorzugt 3.2 bis 4.2. Die Reaktionszeit beträgt 9 bis 15 Stunden, bevorzugt 10 bis 12 Stunden. Dabei wird im Vergleich zur EP-A-0415028 (0.9 molal bezogen auf TAHP-Sulfat) mehr als eine vierfache Raum-Zeit-Ausbeute erzielt.

In dem erfindungsgemäßen Verfahren werden nach beendeter Reaktion Ameisensäure und Wasser abdestilliert, was vorzugsweise unter vermindertem Druck erfolgt. Die so zurückgewonnene wasserhaltige Ameisensäure ist sehr rein und kann in anderen Verfahren eingesetzt werden. Insbesondere kann die zurückgewonnene wasserhaltige Ameisensäure bei der Herstellung des Ausgangsstoffs DAFHP verwendet werden, was einen weiteren Vorteil der vorliegenden Erfindung darstellt.

Die Reinigung des erhaltenen Guanins kann in bekannter Weise erfolgen. Beispielsweise kann das Rohprodukt in wässrigem Alkalihydroxid gelöst und mit Aktivkohle behandelt werden. Anschließend wird Guanin üblicherweise durch Ausfällung, vorzugsweise durch Verseifungsfällung, entsprechend der DE-A-37 23 874, isoliert. Das erfindungsgemäße Verfahren liefert Guanin in sehr guten Ausbeuten von beispielsweise 98% d.Th. als Rohprodukt. Nach Reinigung kann Guanin in guten Ausbeuten von üblicherweise 92% d.Th. und mehr als Endprodukt erhalten werden. Das nach diesem Verfahren gewonnene Reinguanin hat einen Gehalt (HPLC) von mehr als 99.5%.

Das nach dem erfindungsgemäßen Verfahren hergestellte Guanin kann als Zwischenprodukt zur Synthese pharmakologisch wirksamer Verbindungen verwendet werden.

Die nachfolgenden Beispiele sollen die Erfindung näher erläutern, ohne sie jedoch auf diese zu beschränken.

### Beispiel 1

In 222.2 g Ameisensäure (98-100%ig) werden unter Rühren portionsweise 67.6 g (0.4 mol) DAFHP eingetragen (bezogen auf DAFHP beträgt die Molalität 1.8). Das Gemisch wird bis zum Sieden erhitzt, und die gut rührbare Suspension wird anschließend 18 Stunden unter Rückfluß gehalten. Danach werden Ameisensäure und Wasser im Wasserstrahlvakuum nahezu vollständig abdestilliert. Das erhaltene Rohguanin wird in bekannter Weise durch Lösen in wäßrigem Alkalihydroxid, Behandeln mit Aktivkohle (15 Gew.-%) und anschließende Verseifungsfällung gereinigt. Aus 10 g Rohprodukt (aliquoter Teil) werden so nach einmaliger Anwendung dieses Reinigungsverfahrens, 9.61 g (95.9% d.Th.) Guanin mit einem Gehalt (HPLC) von 99.8% gewonnen.

### Beispiel 2

Zu 111.1 g Ameisensäure (98-100%ig) werden unter Rühren zunächst 7.2 g (0.4 mol) Wasser und anschließend portionsweise 67.6 g (0.4 mol) DAFHP gegeben (bezogen auf DAFHP beträgt die Molalitat 3.4). Das Gemisch wird bis zum Sieden erhitzt, und die gut rührbare Suspension wird anschließend 12 Stunden unter Rückfluß gehalten. Danach werden Ameisensaure und Wasser im Wasserstrahlvakuum nahezu vollständig abdestilliert. Die Reinigung erfolgt analog Beispiel 1. Es wird so Guanin mit einem Gehalt (HPLG) von 99.9% in einer Ausbeute von 91.8% d. Th. erhalten.

### Vergleichsbeispiel (nach EP-A-0415028)

Die TAHP-Sulfat-Konzentration in der Reaktionsmischung ist bei diesem Vergleichsbeispiel doppelt so hoch wie in der EP-A-04 15 028.

Ein Gemisch aus 111.1 g Ameisensäure (98-100%ig), 47.8 g (0.2 mol) TAHP-Sulfat und 28.6 g (0.42 mol) Natriumformiat wird bis zum Sieden erhitzt. Nach Erreichen der Rückflußtemperatur wird die Reaktionsmischung dabei unrührbar. Die Rührbarkeit verbessert sich allmählich im Verlauf von mehreren Stunden. Erst nach etwa 5 Stunden liegt eine rührbare Suspension vor. Das Reaktionsgemisch wird insgesamt 18 Stunden unter Rückfluß gehalten. Das nach Abdestillation von Wasser und Ameisensäure im Wasserstrahlvakuum erhaltene Rohprodukt enthält nach HPLC neben 84.2% Guanin noch 14.3% der unumgesetzten Zwischenstufe DAFHP.

## Patentansprüche

1. Verfahren zur Herstellung von Guanin, ausgehend von 2,4-Diamino-5-formylamino-6-hydroxypyrimidin (DAFHP), **dadurch gekennzeichnet**, daß man isoliertes DAFHP in Abwesenheit von Formamid in Ameisensäure mit oder ohne Wasserzusatz unter Rückflußbedingungen zum Guanin umsetzt.

2. Verfahren nach Anspruch 1, **dadurch gekennzeichnet**, daß bezogen auf DAFHP bevorzugt bis zu 2 Äquivalente, besonders bevorzugt 0.5 bis 1.5 Äquivalente Wasser eingesetzt werden.

3. Verfahren nach den Ansprüchen 1 und 2, **dadurch gekennzeichne**t, daß die Reaktionszeit in der Ausführungsform ohne Wasserzusatz 12 bis 24 Stunden, bevorzugt 15 bis 18 Stunden, und in der bevorzugten Ausführungsform mit Wasserzusatz 9 bis 15 Stunden, bevorzugt 10 bis 12 Stunden, beträgt.

4. Verfahren nach den Ansprüchen 1 bis 3, **dadurch gekennzeichnet**, daß die Reaktion bei Normaldruck und bei einer Temperatur, die sich einstellt, wenn wasserhaltige Ameisensäure unter Rückfluß siedet, durchgeführt wird.

5. Verfahren nach den Ansprüchen 1 bis 4, **dadurch gekennzeichnet**, daß die molale Konzentration an DAFHP im Reaktionsgemisch in der Ausführungsform ohne Wasserzusatz bis zu 2.5, bevorzugt 1.80 bis 2.25, und in der bevorzugten Ausführungsform mit Wasserzusatz bis zu 5.0, bevorzugt 3.2 bis 4.2, beträgt.

6. Verfahren nach den Ansprüchen 1 bis 5, **dadurch gekennzeichnet**, daß nach beendeter Reaktion Ameisensäure und Wasser bevorzugt unter vermindertem Druck abdestilliert werden und das so erhaltene Guanin in bekannter Weise in wäßrig alkalischer Lösung mit Aktivkohle gereinigt und nach anschließender Ausfällung, bevorzugt durch Verseifungsfällung, isoliert wird.

7. Verwendung des nach einem der Ansprüche 1 bis 6 hergestellten Guanins als Zwischenprodukt zur Synthese pharmakologisch wirksamer Verbindungen.
